# EUROPÄISCHE PATENTSCHRIFT

(11) **EP 1 527 065 B1**
(45) Veröffentlichungstag und Bekanntmachung des Hinweises auf die Patenterteilung: **22.11.2006**
(21) Anmeldenummer: 03766383.8
(22) Anmeldetag: 31.07.2003
(51) Int. Cl.: C07D 333/20, C07B 57/00

(54) **VERFAHREN ZUR HERSTELLUNG VON (S)-3-METHYLAMINO-1-(THIEN-2-YL)PROPAN-1-OL**
METHOD FOR THE PRODUCTION OF (S)-3-METHYLAMINO-1-(THIEN-2-YL)PROPAN-1-OL
PROCEDE POUR LA PRODUCTION DE (S)-3-METHYLAMINO-1-(THIEN-2-YL)PROPAN-1-OL

(30) Priorität: 01.08.2002 DE 10235206
(43) Veröffentlichungstag der Anmeldung: 04.05.2005
(73) Patentinhaber: BASF Aktiengesellschaft, 67056 Ludwigshafen (DE)
(72) Erfinder: STÜRMER, Rainer, 67127 Rödersheim-Gronau (DE)
(74) Vertreter: Reitstötter - Kinzebach
(86) Internationale Anmeldenummer: PCT/EP2003/008492
(87) Internationale Veröffentlichungsnummer: WO 2004/013123

(56) Entgegenhaltungen:
- EP-A- 0 273 658
- EP-A- 0 457 559
- EP-A- 0 650 965
- US-A- 5 491 243
- PÀMIES, O.; BÄCKVALL, J.E.: "Efficient Lipase-Catalyzed Kinetic Resolution and Dynamic Kinetic Resolution of beta-Hydroxy Nitriles. A Route to Useful Precursors for gamma-Amino Alcohols" ADVANCED SYNTHESIS AND CATALYSIS, Bd. 343, Nr. 6-7, 2001, Seiten 726-731, XP002258894
- DEETER J ET AL: "ASYMMETRIC SYNTHESIS AND ABSOLUTE STEREOCHEMISTRY OF LY248686" TETRAHEDRON LETTERS, ELSEVIER SCIENCE PUBLISHERS, AMSTERDAM, NL, Bd. 31, Nr. 49, 26. November 1990 (1990-11-26), Seiten 7101-7104, XP001119089 ISSN: 0040-4039

## Beschreibung

Die vorliegende Erfindung betrifft ein Verfahren zur Herstellung von enantiomerenreinem (S)-3-Methylamino-1-(thien-2-yl)propan-1-ol der Formel II-S

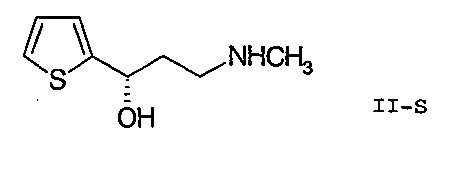

Das Aminopropanol II-S ist eine wichtige Vorstufe für die Synthese des Antidepressivums Duloxetine der Formel IV 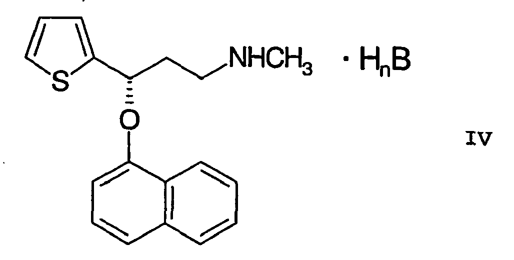 worin B für einen n-fach negativ geladenen anorganischen oder organischen Säurerest steht und HₙB für eine pharmazeutisch verträgliche Säure steht.

Duloxetine ist dementsprechend das Säureadditionssalz des Aminonaphthylethers III (nachfolgend als Duloxetine-Base III bezeichnet)

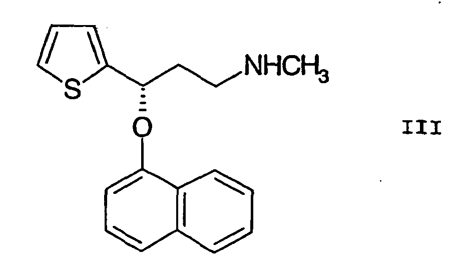

Verfahren des Standes der Technik zur Herstellung der Duloxetine-Base III sind aufwendig und erfordern den Einsatz chiraler Reagenzien oder chiraler Edukte.

So beschreibt die EP-B-0273658 ein Verfahren zur Herstellung der Duloxetine-Base III durch Umsetzung von 2-Acetylthiophen in einer Mannich-Reaktion mit Formaldehyd und Dimethylamin, Reduktion der Ketogruppe der dabei erhaltenen Mannich-Base V zum racemischen (S)-3-N,N-Dimethylamino-1-(thien-2-yl)propan-1-ol VI, Veretherung der Alkoholfunktion mit Naphthylfluorid und schließlich Umwandlung der Dimethylamino-Gruppe in eine Methylamino-Funktion. Das gewünschte Enantiomer des Naphtylethers III erhält man durch Einsatz chiraler Ausgangsmaterialien oder durch Racemattrennung auf der Stufe des Endprodukts, beispielsweise über die Salze mit optisch aktiven Säuren oder Chromatographie an einer chiralen stationären Phase.

Die US-5,362,886 beschreibt ein analoges Verfahren, bei dem der nach Reduktion der Ketogruppe erhaltene racemische Alkohol VI mit S-Mandelsäure versetzt wird. Das hierbei erhaltene S-Enantiomer von VI wird in die nachfolgenden Reaktionsstufen eingesetzt.

Die EP-A-0457559 beschreibt ebenfalls ein der EP-B-0273658 analoges Verfahren. Hierbei wird die Ketogruppe der Mannich-Base V mit dem asymmetrischen Reduktionssystem LAH-lcb (Lithiumaluminiumhydrid-[(2R,2S)-(-)-4-Dimethylamino-1,2-diphenyl-3-methyl-2-butanol]) zum S-Enantiomer von VI reduziert. Nachteilig hierbei ist neben den Kosten die Empfindlichkeit des Reduktionssystems LAH-lcb, das nur wenige Minuten stabil ist.

Aufgabe der vorliegenden Erfindung war die Bereitstellung eines wirtschaftlichen Verfahrens zur Herstellung des Aminoalkohols II-S.

Es wurde überraschenderweise gefunden, dass man den Alkohol II-S enantioselektiv aus Enantiomerengemischen der Alkohole I-S und I-R, insbesondere aus racemischem Alkohol I herstellen kann, indem man zunächst das Enantiomerengemisch in Gegenwart einer Lipase acyliert und das dabei entstehende Acylierungsprodukt des Alkohols I-R vom nicht umgesetzten Alkohol I-S abtrennt. Der Alkohol I-S lässt sich dann in hohen Ausbeuten selektiv durch Reduktion der Nitrilgruppe in Gegenwart von Methylamin in den Alkohol II-S umgewandeln, ohne dass Racemisierung stattfindet.

Die Erfindung betrifft somit ein Verfahren zur Herstellung von enantiomerenreinem (S)-3-Methylamino-1-(thien-2-yl)propan-1-ol der Formel II-S 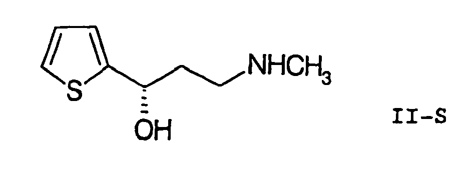 umfassend die folgenden Schritte:
a) Umsetzung eines Enantiomerengemischs der Alkohole (S)-3-Hydroxy-3-thien-2-ylpropionitril und (R)-3-Hydroxy-3-thien-2-ylpropionitril der Formel I-S und I-R 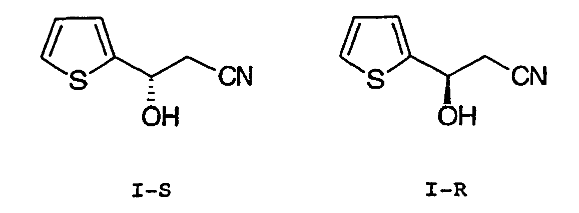 mit einem Acylierungsmittel in Gegenwart einer Lipase, wobei man ein Gemisch von im wesentlichen nicht acyliertem Alkohol I-S und im wesentlichen acyliertem Alkohol I-R erhält;
b) Abtrennung des Alkohols I-S aus dem in Schritt a) erhaltenen Gemisch; und
c) Umsetzung des Alkohols I-S mit Wasserstoff und Methylamin in Gegenwart eines Katalysators zu (S)-3-Methylamino-1-(thien-2-yl)propan-1-ol II-S.

Unter "im wesentlichen nicht acylierter Alkohol I-S" soll verstanden werden, dass wenigstens 95 %, vorzugsweise wenigstens 98 %, insbesondere wenigstens 99 %, des Alkohols I-S nicht acyliert sind. Sinngemäß gilt für den Ausdruck "im wesentlichen acylierter Alkohol I-R", dass wenigstens 95 %, vorzugsweise wenigstens 97 %, besonders bevorzugt wenigstens 98 %, des Alkohols I-R acyliert vorliegen.

Die in Schritt a) eingesetzte Lipase bewirkt eine selektive Veresterung des Alkohols I-R. Die Lipase wird vorzugsweise aus einem Mikroorganismus, besonders bevorzugt aus einem Bakterium oder Pilz, gewonnen. Besonders bevorzugt ist sie bakteriellen Ursprungs. Ebenfalls geeignet sind Lipasen, die durch rekombinante Verfahren erhältlich sind. Die Lipase kann in gereinigter oder teilweise gereinigter Form oder in Form des Mikroorganismus selbst verwendet werden. Verfahren zur Gewinnung und Aufreinigung von Lipasen aus Mikroorganismen sind dem Fachmann hinreichend bekannt, z. B. aus EP 1 149 849 oder EP-A 1 069 183.

Die Lipase kann frei oder immobilisiert eingesetzt werden. Unter einem immobilisierten Enzym versteht man ein Enzym, das an einen inerten Träger fixiert ist. Geeignete Trägermaterialien sowie die darauf immobilisierten Enzyme sind aus der EP-A-1149849, EP-A-1 069 183 und der DE-A-100 19 377 sowie aus den darin zitierten Literaturstellen bekannt. Auf die Offenbarung dieser Schriften wird diesbezüglich in vollem Umfang Bezug genommen. Zu den geeigneten Trägermaterialien gehören beispielsweise Tone, Tonmineralien, wie Kaolinit, Diatomeenerde, Perlit, Siliciumdioxid, Aluminiumoxid, Natriumcarbonat, Calciumcarbonat, Cellulosepulver, Anionenaustauschermaterialien, synthetische Polymere, wie Polystyrol, Acrylharze, Phenolformaldehydharze, Polyurethane und Polyolefine, wie Polyethylen und Polypropylen. Die Trägermaterialien werden zur Herstellung der geträgerten Enzyme üblicherweise in einer feinteiligen, partikelförmigen Form eingesetzt, wobei poröse Formen bevorzugt sind. Die Partikelgröße des Trägermaterials beträgt üblicherweise nicht mehr als 5 mm, insbesondere nicht mehr als 2 mm (Sieblinie). Bevorzugte Trägermaterialien sind mikroporöse Polymerpartikel mit einem Hohlraumanteil von vorzugsweise 40 bis 80 Vol.-% und Porengrößen von vorzugsweise 10 nm bis 1 µm, z. B. teilchenförmiges Polypropylen, das von der Firma Akzo unter der Bezeichnung Accurel^{®} vertrieben wird.

Bevorzugt werden Lipasen (Triacylglycerolacylhydrolasen; EC 3.1.1.3) eingesetzt. Hierunter bevorzugt sind Lipasen, die aus Bakterien der Gattungen Burkholderia oder Pseudomonas gewonnen werden. Beispiele für Burkholderia-Arten sind Burkholderia ambifaria (z. B. Stämme ATCC BAA-244, CCUG 44356, LMG 19182); Burkholderia andropogonis (z. B. Stämme ATCC 23061, CCUG 32772, CFBP 2421, CIP 105771, DSM 9511, ICMP 2807, JCM 10487, LMG 2129, NCPPB 934, NRRL B-14296); Burkholderia caledonica (z. B. Stämme W50D, CCUG 42236, CIP 107098, LMG 19076); Burkholderia caribensis (z. B. Stämme MWAP64, CCUG 42847, CIP 106784, DSM 13236, LMG 18531); Burkholderia caryophylli (z.B. Stämme ATCC 25418, CCUG 20834, CFBP 2429, CFBP 3818, CIP 105770, DSM 50341, HAMBI 2159, ICMP 512, JCM 9310, JCM 10488, LMG 2155, NCPPB 2151); Burkholderia cepacia (z. B. Stämme Ballard 717, 717-ICPB 25, ATCC 25416, CCUG 12691, CCUG 13226, CFBP 2227, CIP 80.24, DSM 7288, HAMBI 1976, ICMP 5796, IFO 14074, JCM 5964, LMG 1222, NCCB 76047, NCPPB 2993, NCTC 10743, NRRL B-14810); Burkholderia cocovenenans (z. B. Stämme ATCC 33664, CFBP 4790, DSM 11318, JCM 10561, LMG 11626, NCIMB 9450); Burkholderia fungorum (z. B. Stämme Croize P763-2, CCUG 31961, CIP 107096, LMG 16225); Burkholderia gladioli (z. B. Stämme ATCC 10248, CCUG 1782, CFBP 2427, CIP 105410, DSM 4285, HAMBI 2157, ICMP 3950, IFO 13700, JCM 9311, LMG 2216, NCCB 38018, NCPPB 1891, NCTC 12378, NRRL B-793); Burkholderia glathei (z. B. Stämme ATCC 29195, CFBP 4791, CIP 105421, DSM 50014, JCM 10563, LMG 14190); Burkholderia glumae (z.B. Stämme ATCC 33617, CCUG 20835, CFBP 4900, CFBP 2430, CIP 106418, DSM 9512, ICMP 3655, LMG 2196, NCPPB 2981, NIAES 1169); Burkholderia graminis (z. B. Stämme C4D1M, ATCC 700544, CCUG 42231, CIP 106649, LMG 18924); Burkholderia kururiensis (z. B. Stämme KP 23, ATCC 700977, CIP 106643, DSM 13646, JCM 10599, LMG 19447); Burkholderia mallei (z. B. Stämme ATCC 23344, NCTC 12938); Burkholderia multivorans (z. B. Stämme ATCC BAA-247, CCUG 34080, CIP 105495, DSM 13243, LMG 13010, NCTC 13007); Burkholderia norimbergensis (z. B. Stämme R2, ATCC BAA-65, CCUG 39188, CFBP 4792, DSM 11628, CIP 105463, JCM 10565, LMG 18379); Burkholderia phenazinium (z. B. Stämme ATCC 33666, CCUG 20836, CFBP 4793, CIP 106502, DSM 10684, JCM 10564, LMG 2247, NCIB 11027); Burkholderia pickettii (z. B. Stämme ATCC 27511, CCUG 3318, CFBP 2459, CIP 73.23, DSM 6297, HAMBI 2158, JCM 5969, LMG 5942, NCTC 11149); Burkholderia plantarii (z. B. Stämme AZ 8201, ATCC 43733, CCUG 23368, CFBP 3573, CFBP 3997, CIP 105769, DSM 9509, ICMP 9424 JCM 5492, LMG 9035, NCPPB 3590, NIAES 1723); Burkholderia pseudomallei (z. B. Stämme WRAIR 286, ATCC 23343, NCTC 12939); Burkholderia pyrrocinia (z. B. Stämme ATCC 15958, CFBP 4794, CIP 105874, DSM 10685, LMG 14191); Burkholderia sacchari (z. B. Stämme CCT 6771, CIP 107211, IPT 101, LMG 19450); Burkholderia solanacearum (z. B. Stämme A. Kelman 60-1, ATCC 11696, CCUG 14272, CFBP 2047, CIP 104762, DSM 9544, ICMP 5712, JCM 10489, LMG 2299, NCAIM B.01459, NCPPB 325, NRRL B-3212); Burkholderia stabilis (z. B. Stämme ATCC BAA-67, CCUG 34168, CIP 106845, LMG 14294, NCTC 13011); Burkholderia thailandensis (z. B. Stämme E 264, ATCC 700388, CIP 106301, DSM 13276); Burkholderia ubonensis (z. B. Stämme EY 3383, CIP 107078, NCTC 13147); Burkholderia vandii (z. B. Stämme VA-1316, ATCC 51545, CFBP 4795, DSM 9510, JCM 7957, LMG 16020); Burkholderia vietnamiensis (z. B. Stämme TW 75, ATCC BAA-248, CCUG 34169, CFBP 4796, CIP 105875, DSM 11319, JCM 10562, LMG 10929). Beispiele für Pseudomonas-Arten sind Pseudomonas aeruginosa (z. B. Stämme ATCC 10145, DSM 50071), Pseudomonas agarici (z. B. Stämme ATCC 25941, DSM 11810), Pseudomonas alcaligenes (z. B. Stämme ATCC 14909, DSM 50342), Pseudomonas amygdali (z. B. Stämme ATCC 337614, DSM 7298), Pseudomonas anguiliseptica (z. B. Stämme ATCC 33660, DSM 12111), Pseudomonas antimicrobica (z. B. Stämme DSM 8361, NCIB 9898, LMG 18920), Pseudomonas aspleni (z. B. Stämme ATCC 23835, CCUG 32773), Pseudomonas aurantiaca (z. B. Stämme ATCC 33663, CIP 106710), Pseudomonas aureofaciens (z. B. Stämme ATCC 13985, CFBP 2133), Pseudomonas avellanae (z. B. Stämme DSM 11809, NCPPB 3487), Pseudomonas azotoformans (z. B. Stämme CIP 106744, JCM 7733), Pseudomonas balearica (z. B. Stämme DSM 6083, CIP 105297)), Pseudomonas beijerinsckii (z. B. Stämme ATCC 19372, DSM 6083), Pseudomonas beteli (z. B. Stämme ATCC 19861, CFBP 4337), Pseudomonas boreopolis (z. B. Stämme ATCC 33662, CIP 106717), Pseudomonas carboxyhydrogena (z. B. Stämme ATCC 29978, DSM 1083), Pseudomonas caricapapayae (z. B. Stämme ATCC 33615, CCUG 32775), Pseudomonas cichorii (z. B. Stämme ATCC 10857, DSM 50259), Pseudomonas cissicola (z. B. Stämme ATCC 33616, CCUG 18839), Pseudomonas citronellolis (z. B. Stämme ATCC 13674, DSM 50332), Pseudomonas coronafaciens (z. B. Stämme DSM 50261, DSM 50262), Pseudomonas corrugata (z. B. Stämme ATCC 29736, DSM 7228), Pseudomonas doudoroffii (z. B. Stämme ATCC 27123, DSM 7028), Pseudomonas echinoides (z. B. Stämme ATCC 14820, DSM 1805), Pseudomonas elongata (z. B. Stämme ATCC 10144, DSM 6810), Pseudomonas ficuserectae (z. B. Stämme ATCC 35104, CCUG 32779), Pseudomonas flavescens (z. B. Stämme ATCC 51555, DSM 12071), Pseudomonas flectens (z. B. Stämme ATCC 12775, CFBB 3281), Pseudomonas fluorescens (z. B. Stämme ATCC 13525, DSM 50090), Pseudomonas fragi (z. B. Stämme ATCC 4973, DSM 3456), Pseudomonas fulva (z. B. Stämme ATCC 31418, CIP 106765), Pseudomonas fuscovaginae (z. B. Stämme CCUG 32780, DSM 7231), Pseudomonas gelidicola (z. B. Stämme CIP 106748), Pseudomonas geniculata (z. B. Stämme ATCC 19374, LMG 2195), Pseudomonas glathei (z. B. Stämme ATCC 29195, DSM 50014), Pseudomonas halophila (z. B. Stämme ATCC 49241, DSM 3050), Pseudomonas hibiscicola (z. B. Stämme ATCC 19867, LMG 980), Pseudomonas huttiensis (z. B. Stämme ATCC 14670, DSM 10281), Pseudomonas iners (z. B. Stamm CIP 106746), Pseudomonas lancelota (z. B. Stämme ATCC 14669, CFBP 5587), Pseudomonas lemoignei (z. B. Stämme ATCC 17989, DSM 7445), Pseudomonas lundensis (z. B. Stämme ATCC 19968, DSM 6252), Pseudomonas luteola (z. B. Stämme ATCC 43273, DSM 6975), Pseudomonas marginalis (z. B. Stämme ATCC 10844, DSM 13124), Pseudomonas meliae (z. B. Stämme ATCC 33050, DSM 6759), Pseudomonas mendocina (z. B. Stämme ATCC 25411, DSM 50017), Pseudomonas mucidolens (z. B. Stämme ATCC 4685, CCUG 1424), Pseudomonas monteilli (z. B. Stämme ATCC 700476, DSM 14164), Pseudomonas nautica (z. B. Stämme ATCC 27132, DSM 50418), Pseudomonas nitroreducens (z. B. Stämme ATCC 33634, DSM 14399), Pseudomonas oleovorans (z. B. Stämme ATCC 8062, DSM 1045), Pseudomonas oryzihabitans (z. B. Stämme ATCC 43272, DSM 6835), Pseudomonas pertucinogena (z. B. Stämme ATCC 190, CCUG 7832), Pseudomonas phenazinium (z. B. Stämme ATCC 33666, DSM 10684), Pseudomonas pictorum (z. B. Stämme ATCC 23328, LMG 981), Pseudomonas pseudoalcaligenes (z. B. Stämme ATCC 17440, DSM 50188), Pseudomonas putida (z. B. Stämme ATCC 12633, DSM 291), Pseudomonas pyrrocinia (z. B. Stämme ATCC 15958, DSM 10685), Pseudomonas resinovorans (z. B. Stämme ATCC 14235, CCUG 2473), Pseudomonas rhodesiae (z. B. Stämme CCUG 38732, DSM 14020), Pseudomonas saccharophila (z. B. Stämme ATCC 15946, DSM 654), Pseudomonas savastanoi (z. B. Stämme ATCC 13522, CFBP 1670), Pseudomonas spinosa (z. B. Stämme ATCC 14606), Pseudomonas stanieri (z. B. Stämme ATCC 27130, DSM 7027), Pseudomonas straminae (z. B. Stämme ATCC 33636, CIP 106745), Pseudomonas stutzeri (z. B. Stämme ATCC 17588, DSM 5190), Pseudomonas synxantha (z. B. Stämme ATCC 9890, CFBP 5591), Pseudomonas syringae (z. B. Stämme ATCC 19310, DSM 6693), Pseudomonas syzygii (z. B. Stämme ATCC 49543, DSM 7385), Pseudomonas taetrolens (z. B. Stämme ATCC 4683, CFBP 5592), Pseudomonas tolaasii (z. B. Stämme ATCC 33618, CCUG 32782), Pseudomonas veronii (z. B. Stämme ATCC 700272, DSM 11331), Pseudomonas viridiflava (z. B. Stämme ATCC 13223, DSM 11124), Pseudomonas vulgaris, Pseudomonas wisconsinensis und Pseudomonas spec. DSM 8246. Davon sind Lipasen aus Burkholderia glumae, Burkholderia plantarii, Burkholderia cepacia, Pseudomonas aeruginosa, Pseudomonas fluorescens, Pseudomonas fragi, Pseudomonas luteola, Pseudomonas vulgaris, Pseudomonas wisconsinensis und Pseudomonas spec. DSM 8246 bevorzugt. Besonders bevorzugt sind Lipasen aus Pseudomonas spec. DSM 8246. In bevorzugten Ausführungsformen werden als Lipasen Enzympräparationen eingesetzt, die aus Pseudomonas spec. DSM 8246 oder Burkholderia plantarii durch Fermentation des Bakteriums und Trocknen des Überstands erhältlich sind; geträgerte Lipasen aus Burkholderia cepacia, die auf Keramik bzw. Diatomit immobilisiert sind und von der Fa. Amano Pharmaceutical Co., Tokyo, Japan unter der Bezeichnung Amano PS-C I, Amano PS-C II bzw. Amano PS-D vertrieben werden; sowie Enzympräparationen aus Burkholderia glumae. Die Fermentation der Bakterien erfolgt beispielsweise wie in der EP-A 1 069 183 beschrieben, auf die hiermit in vollem Umfang Bezug genommen wird.

Die in Schritt a) verwendeten Acylierungsmittel sind beispielsweise Vinyl-, Propenyl- oder Isopropenylester von aliphatischen Monocarbonsäuren mit 2 bis 20 Kohlenstoffatomen, vorzugsweise mit 3 bis 12 Kohlenstoffatomen und besonders bevorzugt mit 3 bis 8 Kohlenstoffatomen. Ebenfalls geeignet sind die Vinyl-, Propenyl- und Isopropenylester von aliphatischen Dicarbonsäuren mit 2 bis 20 Kohlenstoffatomen, bevorzugt von Dicarbonsäuren mit 3 bis 12 Kohlenstoffatomen und besonders bevorzugt mit 4 bis 8 Kohlenstoffatomen. Geeignete Acylierungsmittel sind auch Säureanhydride von aliphatischen Monocarbonsäuren mit 2 bis 12 Kohlenstoffatomen, vorzugsweise mit 3 bis 8 Kohlenstoffatomen, und Säureanhydride von aliphatischen Dicarbonsäuren mit 4 bis 12 Kohlenstoffatomen, vorzugsweise mit 4 oder 5 Kohlenstoffatomen. Beispiele für die Vinyl-, Propenyl- bzw. Isopropenylester von aliphatischen Monocarbonsäuren mit 2 bis 20 Kohlenstoffatomen sind die Vinyl-, Propenyl- bzw. Isopropenylester von Essigsäure, Propionsäure, Buttersäure, Isobuttersäure, Valeriansäure, Isovaleriansäure, 2-Methylbuttersäure, Capronsäure, 2-Methylvaleriansäure, 3-Methylvaleriansäure, 4-Methylvaleriansäure, 2,2-Dimethylbuttersäure, 3,3-Dimethylbuttersäure, Önanthsäure, Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure und Arachinsäure. Unter den vorgenannten Estern sind die Vinylester bevorzugt.

Beispiele für die Vinyl-, Propenyl- bzw. Isopropenylester von aliphatischen Dicarbonsäuren mit 2 bis 20 Kohlenstoffatomen sind die Vinyl-, Propenyl- bzw. Isopropenylester von Oxalsäure, Malonsäure, Bernsteinsäure, Methyl-, Dimethyl-, Trimethyl- und Tetramethylbernsteinsäure, Glutarsäure, 3,3-Dimethylglutarsäure, Adipinsäure, Pimelinsäure, Azelainsäure, Sebacinsäure, Maleinsäure, Fumarsäure und Sorbinsäure. Unter den vorgenannten Estern sind die Vinylester bevorzugt.

Beispiele für die Säureanhydride von aliphatischen Monocarbonsäuren mit 2 bis 12 Kohlenstoffatomen sind die Säureanhydride der oben genannten Carbonsäuren, z. B. diejenigen der Propionsäure, Buttersäure, Isobuttersäure, Valeriansäure, Isovaleriansäure, 2-Methylbuttersäure, Capronsäure, 2-Methylvaleriansäure, 3-Methylvaleriansäure, 4-Methylvaleriansäure, 2,2-Dimethylbuttersäure, 3,3-Dimethylbuttersäure, Önanthsäure und Caprylsäure. Unter den vorgenannten Anhydriden sind diejenigen der Propionsäure, Buttersäure, Valeriansäure und Capronsäure bevorzugt.

Beispiele für die Säureanhydride von aliphatischen Dicarbonsäuren mit 4 bis 12 Kohlenstoffatomen sind Bernsteinsäureanhydrid, Methylbernsteinsäureanhydrid, Dimethylbernsteinsäureanhydrid, Trimethylbernsteinsäureanhydrid, Tetramethylbernsteinsäureanhydrid, Glutarsäureanhydrid und 3,3-Dimethylglutarsäureanhydrid. Hierunter sind Bernsteinsäureanhydrid und Glutarsäureanhydrid bevorzugt.

Unter den in Schritt a) eingesetzten Acylierungsmitteln sind die Vinylester von aliphatischen C₃-C₁₂-Monocarbonsäuren, insbesondere von C₃-C₈-Monocarbonsäuren, wie Propionsäure, Buttersäure, Valeriansäure, Capronsäure, Önanthsäure und Caprylsäure; die Vinylester von aliphatischen C₃-C₁₂-Dicarbonsäuren, insbesondere von C₄-C₈-Dicarbonsäuren, wie Bernsteinsäure, Adipinsäure, Pimelinsäure, Azelainsäure und Sebacinsäure; sowie die Säureanhydride von aliphatischen C₄-C₁₂-Dicarbonsäuren, insbesondere von Dicarbonsäuren mit 4 oder 5 Kohlenstoffatomen, wie Bernsteinsäure und Adipinsäure, bevorzugt. Dies gilt insbesondere dann, wenn man als Enzym eine Lipase, speziell eine Lipase aus einem Bakterium der Gattung Burkholderia oder Pseudomonas, einsetzt.

Vorzugsweise setzt man bei der Umsetzung in Schritt a) 0,6 bis 2 Moläquivalente, besonders bevorzugt 1 bis 1,5 Moläquivalente und insbesondere 1 bis 1,2 Moläquivalente des Acylierungsmittels, bezogen auf den Gehalt an Alkohol I-R im Enantiomerengemisch, ein. Unter Moläquivalenten soll die Anzahl der Carboxylgruppen des Acylierungsmittels in mol verstanden werden, die mit 1 mol Alkohol I-R reagieren können. Entsprechend setzt man bei der Verwendung von Vinyl-, Propenyl- oder Isopropenylestern von aliphatischen Monocarbonsäuren sowie von Säureanhydriden von aliphatischen Mono- oder Dicarbonsäuren vorzugsweise 1,6 bis 2 mol, besonders bevorzugt 1 bis 1,5 mol und insbesondere 1 bis 1,2 mol des Acylierungsmittels, bezogen auf 1 mol des im Enantiomerengemisch enthaltenen Alkohols I-R, ein, während man bei der Verwendung von Vinyl-, Propenyl- oder Isopropenylestern von aliphatischen Dicarbonsäuren vorzugsweise 0,3 bis 1 mol, besonders bevorzugt 0,5 bis 0,8 mol und insbesondere 0,5 bis 0,6 mol Acylierungsmittel pro mol I-R einsetzt.

In einer bevorzugten Ausführungsform wird die Umsetzung in Schritt a) in einem nicht-wässrigen Reaktionsmedium durchgeführt. Unter nicht-wässrigen Reaktionsmedien sollen Reaktionsmedien verstanden werden, die weniger als 1 Gew.-%, vorzugsweise weniger als 0,5 Gew.-% Wasser, bezogen auf das Gesamtgewicht des Reaktionsmediums, enthalten. Vorzugsweise wird die Umsetzung in einem organischen Lösungsmittel durchgeführt. Geeignete Lösungsmittel sind beispielsweise aliphatische Kohlenwasserstoffe, vorzugsweise mit 5 bis 8 Kohlenstoffatomen, wie Pentan, Cyclopentan, Hexan, Cyclohexan, Heptan, Octan oder Cyclooctan, halogenierte aliphatische Kohlenwasserstoffe, vorzugsweise mit einem oder zwei Kohlenstoffatomen, wie Dichlormethan, Chloroform, Tetrachlorkohlenstoff, Dichlorethan oder Tetrachlorethan, aromatische Kohlenwasserstoffe, wie Benzol, Toluol, die Xylole, Chlorbenzol oder Dichlorbenzol, aliphatische acyclische und cyclische Ether, vorzugsweise mit 4 bis 8 Kohlenstoffatomen, wie Diethylether, Methyl-tert-butylether, Ethyl-tert-butylether, Dipropylether, Diisopropylether, Dibutylether, Tetrahydrofuran oder Dioxan oder Gemische davon. Besonders bevorzugt werden die vorgenannten Ether, insbesondere Tetrahydrofuran, verwendet.

Die Reaktanden werden vorzugsweise in einer Konzentration von 1 g/l bis 500 g/l insbesondere von 100 g/l bis 500 g/l eingesetzt.

In einer weiteren bevorzugten Ausführungsform des erfindungsgemäßen Verfahrens erfolgt die Umsetzung in Schritt a) in Substanz, d. h. ohne wässriges oder organisches Lösungsmittel. In diesem Fall ist das Acylierungsmittel vorzugsweise ausgewählt unter den oben genannten Vinyl-, Propenyl- oder Isopropenylestern, insbesondere den Vinylestern.

Die Umsetzung in Schritt a) erfolgt in der Regel bei einer Reaktionstemperatur unterhalb der Desaktivierungstemperatur der eingesetzten Hydrolase und vorzugsweise bei wenigstens -10 °C. Besonders bevorzugt liegt sie im Bereich von 0 bis 100 °C, insbesondere von 20 bis 60 °C und speziell von 20 bis 40 °C. Besonders bevorzugt wird die Umsetzung bei der Temperatur, bei der die Hydrolase ihre höchste Aktivität besitzt, durchgeführt.

Zur Durchführung kann man beispielsweise das Gemisch der Alkohole I-R und I-S mit der Lipase, dem Acylierungsmittel und gegebenenfalls dem Lösungsmittel vorlegen und das Gemisch durchmischen, z. B. durch Rühren oder Schütteln. Es ist aber auch möglich, die Hydrolase in einem Reaktor, beispielsweise in einer Säule, zu immobilisieren, und durch den Reaktor eine den Alkohol I und das Acylierungsmittel enthaltende Mischung zu leiten. Hierzu kann man die Mischung im Kreislauf durch den Reaktor leiten bis der gewünschte Umsatz erreicht ist. Dabei werden die Carboxylgruppen des Acylierungsmittels sequentiell in Ester des R-Enantiomers überführt, während das S-Enantiomer im Wesentlichen unverändert bleibt. In der Regel wird man die Veresterung in Schritt a) bis zu einem Umsatz von wenigstens 95 %, besonders bevorzugt von wenigstens 99 % und insbesondere von wenigstens 99,5 %, bezogen auf den in der Mischung enthaltenen Alkohol I-R führen. Das Fortschreiten der Reaktion, d. h. die sequentielle Veresterung des Alkohols I-R, kann dabei durch übliche Methoden wie Gaschromatographie verfolgt werden.

Bei dem eingesetzten Enantiomerengemisch der Alkohole I-R und I-S handelt es sich vorzugsweise um deren Racemat.

Die Aufarbeitung des Reaktionsgemischs kann in üblicher Weise erfolgen, beispielsweise, indem man zunächst die Lipase aus dem Reaktionsgemisch abtrennt, z. B. durch Abfiltrieren oder Abzentrifugieren , gegebenenfalls aus dem Filtrat bzw. Zentrifugat das Lösungsmittel entfernt und den Rückstand anschließend einer Trennoperation unterwirft. Geeignete Trennoperationen sind beispielsweise Extraktion, Destillation, Kristallisation oder Chromatographie, wobei man die Trennoperation in Abhängigkeit vom verwendeten Acylierungsmittel auswählt. So trennt man ein Reaktionsgemisch, das bei der Umsetzung von I mit Vinyl-; Propenyl- oder Isopropenylestern von aliphatischen Dicarbonsäuren erhalten wird, vorzugsweise destillativ, während man ein Reaktionsgemisch, bei dem in Schritt a) Vinyl-, Propenyl- oder Isopropenylester von aliphatischen Monocarbonsäuren, Säureanhydride von aliphatischen Monocarbonsäuren oder Säureanhydride von aliphatischen Dicarbonsäuren als Acylierungsmittel eingesetzt wurden, vorzugsweise extraktiv trennt.

Es ist aber auch möglich, das gesamte Reaktionsgemisch aus Schritt a), gegebenenfalls unter vorheriger Abtrennung des Lösungsmittels, direkt der Trennoperation zu unterwerfen, wobei jedoch die vorherige Abtrennung des Enzyms bevorzugt ist.

Der Enantiomerenüberschuss des in Schritt b) abgetrennten Alkohols I-S kann mittels gängiger Verfahren bestimmt werden, beispielsweise durch Bestimmung der optischen Drehung oder durch Chromatographie an einer chiralen Phase, beispielsweise durch HPLC oder Gaschromatographie über chirale Säulen.

Mit dem erfindungsgemäßen Verfahren wird der Alkohol I-S mit einem Enantiomerenüberschuss (ee-Wert) von vorzugsweise wenigstens 98 %, besonders bevorzugt von wenigstens 99 % und insbesondere von wenigstens 99,4 % erhalten.

Die Enantiomerenreinheit des Acylierungsprodukts von I-R kann mit den gleichen Verfahren bestimmt werden. Vorzugsweise beträgt der ee-Wert wenigstens 97 %, besonders bevorzugt wenigstens 98 %.

Eine weitere bevorzugte Ausgestaltung des erfindungsgemäßen Verfahrens umfasst zusätzlich die Schritte:
b1) Gewinnung eines an Alkohol I-R angereicherten Enantiomeren gemischs aus dem nach Abtrennung des Alkohols I-S erhaltenen Rückstands durch Hydrolyse des Rückstands,
b2) Racemisierung dieses Enantiomerengemischs und
b3) Rückführung des Racemats in Schritt a).

Die Hydrolyse des acylierten Alkohols I-R in Schritt b1) kann nach gängigen Verfahren erfolgen, beispielsweise durch Umsetzung mit einer Base. Geeignete Basen sind beispielsweise Alkali- und Erdalkalihydroxide und -carbonate, Ammoniak, Amine, wie Dimethylamin, Diethylamin, Trimethylamin, Triethylamin und Diisopropylamin. Vorzugsweise verwendet man zur Hydrolyse als Base Natrium- oder Kaliumhydroxid. Die Hydrolyse kann in Wasser oder in einem Lösungsmittel oder in einer Mischung aus Wasser und Lösungsmittel durchgeführt werden. Geeignete Lösungsmittel sind Alkohole, vorzugsweise mit 1 bis 3 Kohlenstoffatomen, wie Methanol, Ethanol, Propanol oder Isopropanol, Glykole, insbesondere mit 2 bis 8 Kohlenstoffatomen, wie Ethylenglykol, Di- und Triethylenglykol, die Gemische der vorstehend genannten Lösungsmittel sowie deren Gemische mit Wasser.

Die Racemisierung des in Schritt b1) gewonnenen Enantiomerengemischs aus in Schritt b) nicht vollständig abgetrenntem Alkohol I-R und durch Hydrolyse des Acylierungsprodukts gewonnenem Alkohol I-S kann ebenfalls mittels bekannter Verfahren erfolgen, beispielsweise durch Umsetzung mit einer Säure oder vorzugsweise durch Oxidation der Alkoholfunktion zu einer Ketogruppe und deren Reduktion zur Alkoholfunktion. Geeignete Oxidatinsmittel sind dem Fachmann bekannt. Dazu zählen beispielsweise Braunstein, Wasserstoffperoxid, Wolfram(VI)oxid·H₂O₂, aktivierte DMSO-Systeme (nach Corey oder Swern),enzymatische Systeme (Dehydrogenasen) u.a. Geeignete Reduktionsmittel sind dem Fachmann ebenfalls bekannt und umfassen beispielsweise Natriumborhydrid und Wasserstoff (katalytische Hydrierung).

Geeignete Katalysatoren für die Umsetzung in Schritt c) sind vorzugsweise ausgewählt unter Heterogenkatalysatoren, die wenigstens ein Metall der Nebengruppe VIII umfassen, oder unter Homogenkatalysatoren, die wenigstens ein Metall der Nebengruppe VIII und wenigstens einen phosphorhaltigen Liganden umfassen.

Bevorzugte Heterogenkatalysatoren umfassen als Metalle der Nebengruppe VIII Platin, Palladium, Nickel, Ruthenium und/oder Rhodium, wobei die Metalle in elementarer oder oxidischer Form eingesetzt werden können.

Das Metall kann zur Erhöhung der Aktivität und/oder Stabilität auf einen Träger aufgebracht sein. Geeignete Trägermaterialien umfassen sowohl metallische als auch nichtmetallische Materialien, wobei diese sowohl porös als auch nichtporös sein können. Metallische Träger bestehen vorzugsweise aus hochlegierten Edelstählen. Poröse nichtmetallische Träger bestehen vorzugsweise aus Aktivkohle, Silikaten, Aluminiumoxid, Tonerden oder Zeolithen. Nichtporöse nichtmetallische Träger bestehen vorzugsweise aus mineralischen Werkstoffen, wie natürliche oder synthetische Mineralien, Gläser und Keramiken, aus Kunststoffen oder aus einer Kombination von beiden. Der Metallgehalt im geträgerten Heterogenkatalysator beträgt in der Regel 0,001 bis 10 Gew.-%, häufig 0,1 bis 7 Gew.-%, bezogen auf das Gewicht des Trägers. Vorzugsweise ist der Träger ausgewählt unter den oben genannten porösen nichtmetallischen Trägern, insbesondere unter Aktivkohle, Silikaten und Aluminiumoxid.

Bei der Verwendung von nichtgeträgerten Metallen werden diese, insbesondere Nickel, vorzugsweise in Form von Metallschwämmen, als sogenannte Raney-Katalysatoren, eingesetzt.

Bevorzugte Heterogenkatalysatoren sind elementares Palladium und Nickel, besonders bevorzugt Nickel und speziell Raney-Nickel.

Die Metalle der Nebengruppe VIII der Homogenkatalysatoren sind vorzugsweise ausgewählt unter Palladium, Nickel, Platin, Ruthenium und Rhodium und besonders bevorzugt unter Ruthenium und Rhodium.

Bei den phosphorhaltigen Liganden der Homogenkatalysatoren handelt es sich vorzugsweise um PF₃, Phosphole, Phosphabenzole und um 1-, 2- und mehrzähnige Phosphin-, Phosphinit-, Phosphonit-, Phosphoramidit- und Phosphitliganden. Das Phosphoratom ist in der Regel mit wenigstens einer Alkyl-, Cycloalkyl-, Heterocycloalkyl-, Aryl-, Arylalkyl-, Heteroaryl- oder Heteroarylalkylgruppe substituiert, die ihrerseits durch eine oder mehrere dieser Gruppen oder durch einen der Phosphor-haltigen Liganden substituiert sein kann. Zwei oder mehrere Gruppen können auch gemeinsam eine verbrückende Einheit bilden.

Die Alkylreste am Phophoratom enthalten insbesondere 1 bis 20 und speziell 1 bis 8 Kohlenstoffatome. Sie können verzweigt oder unverzweigt vorliegen. Beispiele dafür sind Methyl, Ethyl, n-Propyl, Isopropyl, n-Butyl, sec-Butyl, Isobutyl, tert-Butyl, n-Pentyl, 2- und 3-Pentyl, 2- und 3- Methylbutyl, Neopentyl, n-Hexyl, 2-, 3- und 4-Hexyl, 2-, 3- und 4-Methylpentyl, 2-Ethylbutyl, n-Heptyl, n-Octyl und 2-Ethylhexyl.

Bei der Cycloalkylgruppe handelt es sich vorzugsweise um eine C₅-C₇-Cycloalkylgruppe wie Cyclopentyl, Cyclohexyl und Cycloheptyl.

Die Heterocycloalkylgruppe enthält in der Regel 2 bis 6 Kohlenstoffatome sowie 1 bis 3 Heteroatome, die ausgewählt sind unter Sauerstoff, Schwefel, einfach substituiertem Stickstoff und zweifach substituiertem Silicium. Beispiele hierfür sind Tetrahydrofuran, Di- und Tetrahydropyran, Dioxan, Pyrrolidin, Piperidin, Piperazin und Morpholin.

Aryl steht beispielsweise für Phenyl, Tolyl, Xylyl, Mesityl, Naphthyl, Anthranyl, Phenanthryl, Naphthacenyl, vorzugsweise für Phenyl oder Naphthyl und insbesondere für Phenyl.

Heteroaryl steht beispielsweise für Pyrrolyl, Pyrazolyl, Imidazolyl, Indolyl, Carbazolyl, Pyridyl, Chinolinyl, Acridinyl, Pyridazinyl, Pyrimidinyl oder Pyrazinyl.

Die obigen Ausführungen und Bevorzugungen gelten entsprechend für die Arylalkyl- und Heteroarylalkylsubstituenten.

Bei mehrzähnigen Phosphor-haltigen Liganden mit wenigstens zwei Phosphoratomen sind diese vorzugsweise über C₁-C₅-, insbesondere über C₂-C₄-Einheiten, die gesättigt oder ungesättigt sein können, verbrückt.

Bevorzugte Phosphor-haltige Liganden sind Tris(triphenylphosphin) und Tris(tritolylphosphin).

Bevorzugte Homogenkatalysatoren sind Rhodium-tris(triphenylphosphin)-chlorid (RhCl(PPh₃)₃; Wilkinson-Katalysator) und Ruthenium-bis(triphenylphosphin)-dichlorid (RuCl₂(PPh₃)₃.

Neben den Phosphor-haltigen Liganden enthält der Metallkomplex gegebenenfalls noch zusätzlich Liganden, z. B. Halogenide, Amine, Carboxylate, Sulfonate, Hydrid, CO, Olefinen, Diene, Nitrile, stickstoffhaltige Heterocylen, Aromaten und Ether.

Der Katalysator wird vorzugsweise in einer Menge von 0,1 bis 5 Mol-% Metall, bezogen auf die Menge des eingesetzten Alkohols I-S, eingesetzt.

Der Katalysator kann auch als Prä-Katalysator eingesetzt werden, d. h. der eigentliche Katalysator wird erst *in situ* durch Umsetzung des Prä-Katalysators mit einem Phosphor-haltigen Liganden erzeugt.

Bei der Hydrierung in Schritt c) verwendet man vorzugsweise einen Heterogenkatalysator, besonders bevorzugt einen Nickel- oder Palladiumkatalysator, wobei Palladium vorzugsweise auf Kohle geträgert eingesetzt wird, und insbesondere Raney-Nickel.

Die Umsetzung in Schritt c) wird vorzugsweise in einem geeigneten Lösungsmittel durchgeführt.

Bevorzugte Lösungsmittel sind C₁-C₄-Alkohole, wie Methanol, Ethanol, Propanol, Isopropanol, Butanol oder tert-Butanol, aliphatische acyclische oder cyclischen Ether, wie Diethylether, Dipropylether, Methyl-tert-butylether, Tetrahydrofuran oder Dioxan, Wasser oder deren Gemische.

Das in Schritt c) eingesetzte Methylamin kann entweder gasförmig oder als wässrige Lösung eingesetzt werden, wobei es vorzugsweise als wässrige Lösung verwendet wird. Vorzugsweise wird es in einer Menge von 1 - 100 Moläquivalenten, besonders bevorzugt von 10 - 20 Moläquivalenten, bezogen auf die Menge des eingesetzten Alkohols I-S, eingesetzt.

Die Reaktion in Schritt c) wird vorzugsweise bei einem Druck von 1 bis 250 bar, besonders bevorzugt von 10 bis 200 bar, insbesondere von 50 bis 150 bar, durchgeführt. Die Reaktionstemperatur beträgt vorzugsweise 25 bis 140 °C, besonders bevorzugt 40 bis 100 °C.

Die Aufarbeitung der Reaktion kann in üblicher Weise erfolgen, beispielsweise, indem man den Katalysator gegebenenfalls zuerst desaktiviert und anschließend abtrennt, das Lösungsmittel entfernt und aus dem Rückstand sauberes II-S beispielsweise durch Kristallisation, Destillation, Extraktion oder Chromatographie isoliert.

Durch das erfindungsgemäße Verfahren erhält man das Methylaminopropanol II-S mit einem Enantionmerenüberschuß (ee-Wert) von wenigstens 98 %, vorzugsweise von wenigstens 99 % und insbesondere von wenigstens 99,4 %.

Die Herstellung eines in Schritt a) eingesetzten Enantiomerengemischs der Alkohole I-S und I-R gelingt beispielsweise durch Umsetzung von Thiophen-2-carbaldehyd mit Acetonitril in Gegenwart einer Base gemäß dem folgenden Schema: 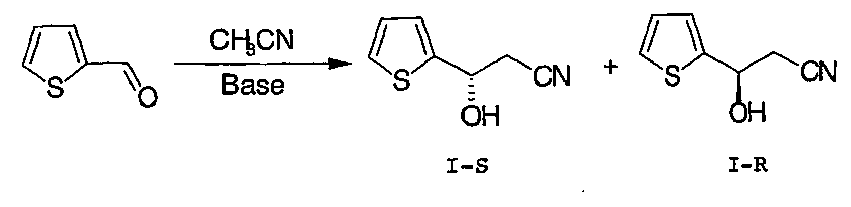 oder durch nucleophile Ringöffnung von 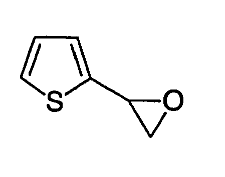 mit Cyanhydrin analog der in US 5,136,078 beschriebenen Methode.

Als Base für die Kondensation von Thiophen-2-carbaldehyd mit Acetonitril verwendet man vorzugsweise Alkali- und Erdalkalihydroxide, wie Natriumhydroxid oder Kaliumhydroxid, Alkalihydride, wie Natriumhydrid, Alkalialkoholate, wie Natriummethanolat, Natriumethanolat, Natrium-tert-butylat und Kalium-tert-butylat und Amine, wie Dimethylamin, Triethylamin, Diethylamin, Triethylamin oder Diisopropylamin. Besonders bevorzugt verwendet man Kalium- oder Natrium-tert-butylat.

Als Lösungsmittel verwendet man vorzugsweise polar-aprotische Lösungsmittel, wie Tetrahydrofuran (THF), Dioxan, Dimethylformamid oder Dimethylsulfoxid, wobei THF besonders bevorzugt ist. Bei der Verwendung von Alkalihydroxiden als Base ist es jedoch auch möglich, unter Phasentransferbedingungen zu arbeiten, wobei man in diesem Fall als organisches Lösungsmittel vorzugsweise aliphatische acyclische Ether, wie Diethylether, Diisopropylether, Dipropylether oder Methy-tert-butylether verwendet, wobei man das Alkalihydroxid in der wässrigen Phase vorlegt.

Die Umsetzung des Thiophen-2-carbaldehyds erfolgt vorzugsweise bei einer Reaktionstemperatur von -78 °C bis 50 °C, besonders bevorzugt von -10 °C bis 30 °C und speziell bei Raumtemperatur. Die Durchführung erfolgt vorzugsweise in der Weise, dass man die Base im Lösungsmittel vorlegt, auf die gewünschte Reaktionstemperatur abkühlt und anschließend nacheinander Acetonitril und Thiophen-2-carbaldehyd zugibt. Es ist auch möglich, zuerst Thiophen-2-carbaldehyd und danach Acetonitril zuzugeben, wobei jedoch die erste Vorgehensweise bevorzugt ist. Die Aufarbeitung des Reaktionsgemischs kann in üblicher Weise erfolgen, beispielsweise durch Versetzen des Reaktionsgemischs mit Wasser, Extraktion der wässrigen Phase mit einem geeigneten organischen Lösungsmittel und Gewinnung des Alkohols aus dem organischen Extrakt. Zur Extraktion geeignete Lösungsmittel sind beispielsweise acyclische Ether, vorzugsweise mit 4 bis 8 Kohlenstoffatomen, wie Diethylether, Dipropylether, Diisopropylether, Methyl-tert-butylether, Ethyl-tert-butylether und Dibutylether, Ester, vorzugsweise mit 3 bis 8 Kohlenstoffatomen wie Essigsäuremethyl- und -ethylester, aliphatische Kohlenwasserstoffen, vorzugsweise mit 5 bis 8 C-Atomen, wie Pentan, Cyclopentan, Hexan, Cyclohexan, Heptan, Cycloocten, aromatische Kohlenwasserstoffe, wie Benzol, Toluol, die Xylole, Chlorbenzol, Dichlorbenzol, und halogenierte aliphatische Kohlenwasserstoffe, vorzugsweise mit 1 oder 2 Kohlenstoffatomen, wie Dichlormethan, Trichlormethan, Tetrachlorkohlenstoff, Dichlorethan oder Tetrachlorethan. Vorzugsweise verwendet man einen der oben genannten Ether, insbesondere Diethylether oder Methyl-tert-butylether.

Durch das erfindungsgemäße Verfahren gewinnt man den Aminoalkohol II-S ohne teure Reagenzien und ohne aufwendige Reaktionsbedingungen in hoher optischer Reinheit.

Die folgenden Beispiele dienen der Veranschaulichung der Erfindung, sind jedoch nicht einschränkend zu verstehen.

### Beispiele:

### 1. Herstellung des Enantiomerengemischs der Alkohole I-R und I-S

### 1.1 Umsetzung bei -50 °C

In einem Kolben mit Rührung und externer Kühlung wurden 22,0 g (196,4 mmol) Kalium-tert-butanolat in 150 ml Tetrahydrofuran (THF) vorgelegt, auf -50 °C abgekühlt und innerhalb von 10 Minuten wurden 8,05 g (196,4 mmol) Acetonitril zugetropft. Nach einer Stunde Rühren bei -50 °C gab man 20,0 g (178,6 mmol) Thiophen-2-carbaldehyd zu. Man ließ das Gemisch auf Raumtemperatur auftauen, versetzte es mit 100 ml Wasser, trennte die organische Phase ab und extrahierte die wässrige Phase mit 100 ml Methyl-tert-butylether (MTBE). Die vereinigten organischen Extrakte wurden über Natriumsulfat getrocknet und das Lösungsmittel wurde bei vermindertem Druck destillativ entfernt. Man erhielt 24,8 g (91 % der Theorie) des Enantiomerengemischs als schwachgelbes Öl mit einer Reinheit (GC) von mehr als 95 %.
¹H-NMR (400 MHz; CDCl₃): 7,25 (m, 1H), 7,02 (m, 1H), 6,92 (m, 1H), 5,10 (t, 1H), 4,10 (br s,1H), 2,75 (d, 2H).

### 1.2 Umsetzung bei Raumtemperatur

In einem Kolben mit Rührung und Innenthermometer wurden 260 g (2,71 mol) Natrium-tert-butanolat in 2 1 Tetrahydrofuran (THF) vorgelegt und innerhalb von 30 Minuten wurden 110 g (2,71 mol) Acetonitril zugetropft. Dabei wurde darauf geachtet, dass die Innentemperatur 35 °C nicht überschritt. Nach beendigter Zugabe wurde 30 Minuten bei Raumtemperatur nachgerührt. Anschließend gab man 280 g (2,5 mol) Thiophen-2-carbaldehyd innerhalb einer Stunde zu. Man ließ das Gemisch 2,5 Stunden bei Raumtemperatur nachrühren, versetzte es dann mit 1,5 1 Wasser und 1,5 1 Methyl-tert-butylether (MTBE), trennte die organische Phase ab und extrahierte die wässrige Phase mit 100 ml MTBE. Die vereinigten organischen Extrakte wurden über Natriumsulfat getrocknet und das Lösungsmittel wurde bei vermindertem Druck destillativ entfernt. Man erhielt 389 g (100 % der Theorie) des rohen Enantiomerengemischs als hellbraunes Öl mit einer Reinheit (GC) von mehr als 97 %.

### 2. Gewinnung von I-S

### 2.1 Gewinnung von I-S durch Umsetzung in MTBE

Die Lipase-haltige Enzympräparation aus Pseudomonas spec. DSM 8246 wurde analog dem in der EP-A 1 069 183 beschriebenen Beispiel 1.1 hergestellt, wobei die Enzympräparation durch Sprühtrocknen des Fermentationsüberstands erhalten wurde.

5,00 g (32 mmol) des Enantiomerengemischs aus Beispiel 2 wurden in 20 ml Methyl-tert-butylether (MTBE) vorgelegt und mit 19 mmol des Acylierungsmittels sowie mit 0,1 g Lipase-haltige Enzympräparation aus Pseudomonas spec. DSM 8246 versetzt. Das Gemisch wurde 6 Stunden bei Raumtemperatur geschüttelt. Anschließend wurde das Enzym abfiltriert, das Filtrat eingeengt und der Rückstand flash-chromatographiert (Kieselgel 60; Hexan/Ethylacetat 1:1)). Man erhielt 2,40 g (48 % der Theorie) des Alkohols I-S. Die Acylierungsmittel und die damit erhaltenen Enantiomerenüberschüsse sind in nachfolgender Tabelle aufgeführt. Der Enantiomerenüberschuss wurde gaschromatographisch mittels einer chiralen Säule (Säule: GTA der Firma Chiraldex, 20 m x 0,25 mm; Splitbetrieb; Carrier: 70 kpa Helium) bestimmt.

| **Acylierungsmittel** | **ee-Wert [%]** |
|---|---|
| Vinylhexanoat | 99,4 |
| Bernsteinsäureanhydrid | 99,5 |
| Vinylpropionat | 99,6 |

Der Enantionmerenüberschuss des acylierten Alkohols I-R betrug jeweils 98 %.

### 2.2 Gewinnung von I-S durch lösungsmittelfreie Umsetzung

Die Versuchsdurchführung entsprach derjenigen des Beispiels 2.1, wobei jedoch kein Methyl-tert-butylether verwendet wurde. Als Acylierungsmittel verwendete man 19 mmol Vinylhexanoat. Die Abtrennung des Enzyms erfolgte durch Filtration. Man erhielt den Alkohol I-S in einer Ausbeute von 2,40 g (48 % der Theorie) mit einem Enantiomerenüberschuss von 99,6 %.

### 2.3 Gewinnung von I-S in Gegenwart anderer Enzyme und/oder anderer Lösungsmittel

Die Versuchsdurchführung entsprach derjenigen aus Beispiel 2.1, wobei man jedoch das Enzym und/oder das Lösungsmittel variierte. Als Acylierungsmittel setzte man Bernsteinsäureanhydrid ein. Die nachfolgende Tabelle zeigt die erzielten ee-Werte des gewonnen Alkohols I-S nach 50%igem Umsatz.

| Lipase | Lösungsmittel | ee-Wert [%] |
|---|---|---|
| Pseudomonas spec.¹ | Isooctan | 92 |
| Amano PS-C I² | MTBE⁴ | 88 |
| Amano PS-C II² | MTBE⁴ | 92 |
| Amano PS-D³ | MTBE⁴ | 91 |

| | | |
|---|---|---|
| ¹ Pseudomonas spec. DSM 8246 ² Produktbezeichnung der Fa. Amano Pharmaceutical Co., Tokyo, Japan: Enzym aus Burkholderia cepacia; auf Keramik immobilisiert ³ Produktbezeichnung der Fa. Amano Pharmaceutical Co., Tokyo, Japan: Enzym aus Burkholderia cepacia; auf Diatomit immobilisiert ⁴ Methyl-tert-butylether | | |

### 3. Herstellung des Aminoalkohols II-S

### 3.1 Verwendung von Palladium auf Kohle als Katalysator

In einem Laborautoklaven legte man 2,40 g (15,7 mmol) des Alkohols I-S mit einem ee-Wert von 99,4 % in 10 ml Methanol vor, versetzte die Lösung mit 10 Moläquivalenten wässrigen Methylamins (40%ig in Wasser) und mit 25 mg 5 % Palladium auf Kohle (von der Fa. Degussa) und hydrierte das Reaktionsgemisch bei 60 °C und 100 bar Wasserstoffdruck 24 Stunden. Anschließend filtrierte man den Katalysator ab, verdampfte das Lösungsmittel im Vakuum und kristallisierte den Rückstand aus Cyclohexan/Isopropanol um. Man erhielt 1,98 g (74 % der Theorie) des Aminoalkohols II-S als farblosen Feststoff.
Drehwert: [α]_{D} = -12,14° bei 28°C.
Enantiomerenüberschuss (Bestimmung siehe 2.1): > 99,5 %
¹H-NMR (400 MHz, CDCl₃): 7.2 (d,1H); 6.9 (m,1H); 5.1 (dd,1H); 4.1 (br s,1H); 2.7 (m,2H); 2.4 (s,3H); 1.9 (m,2H).

### 3.2 Verwendung von Raney-Nickel als Katalysator

In einem Laborautoklaven legte man 28,8 g (0,19 mol) des Alkohols I-S mit einem ee-Wert von 99,4 % in 120 ml Methanol vor, versetzte die Lösung mit 12 Moläquivalenten wässrigen Methylamins (40%ig in Wasser) und mit 0,6 g gewaschenem Raney-Nickel (Raney-Nickel W-02 der Fa. Degussa) und hydrierte das Reaktionsgemisch bei 65 °C und 50 bar Wasserstoffdruck 24 Stunden. Anschließend filtrierte man den Katalysator ab, verdampfte das Lösungsmittel im Vakuum und kristallisierte den Rückstand aus Cyclohexan/Isopropanol (10:1) um. Man erhielt 25,36 g (79 % der Theorie) des Aminoalkohols II-S als farblosen Feststoff.
Drehwert: [α]_{D} = -12,54° bei 25°C.
Enantiomerenüberschuss (Bestimmung siehe 2.1): > 99,5 %
Schmelzpunkt: 69 °C

## Patentansprüche

1. verfahren zur Herstellung von enantiomerenreinem (S)-3-Methylamino-1-(thien-2-yl)propan-1-ol der Formel II-S 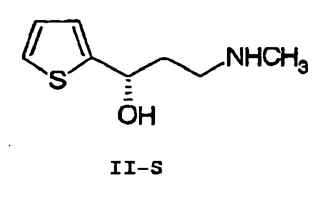 umfassend die folgenden Schritte:
a) Umsetzung eines Enantiomerengemischs der Alkohole (S)-3-Hydroxy-3-thien-2-ylpropionitril und (R)-3-Hydroxy-3-thien-2-ylpropionitril der Formel I-S und I-R 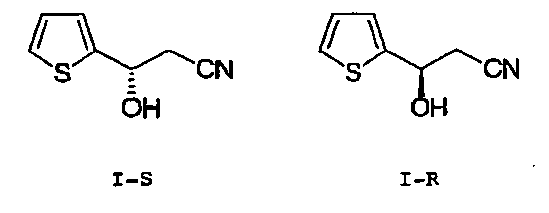 mit einem Acylierungsmittel in Gegenwart einer Lipase, wobei man ein Gemisch von im wesentlichen nicht acyliertem Alkohol I-S und im wesentlichen acyliertem Alkohol I-R erhält;
b) Abtrennung des Alkohols I-S aus dem in Schritt a) erhaltenen Gemisch; und
c) Umsetzung des Alkohols I-S mit Wasserstoff und Methylamin in Gegenwart eines Katalysators zu (S)-3-Methylamino-1-(thien-2-yl)propan-1-ol II-S.

2. verfahren nach Anspruch 1, wobei die Lipase in Schritt a) ausgewählt ist unter Lipasen aus Bakterien der Gattungen Burkholderia oder Pseudomonas.

3. Verfahren nach Anspruch 2, wobei die Lipase eine Lipase aus Burkholderia plantarii, Burkholderia cepacia, Burkholderia glumae, Pseudomonas aeruginosa, Pseudomonas fluorescens, Pseudomonas fragi, Pseudomonas luteola, Pseudomonas vulgaris, pseudomonas wisconsinensis und Pseudomonas spec. DSM 8246 ist.

4. Verfahren nach einem der vorhergehenden Ansprüche, wobei das Acylierungsmittel ausgewählt ist unter den Vinyl-, Propenyl- oder Isopropenylestern von aliphatischen Monocarbonsäuren mit 3 bis 12 Kohlenstoffatomen und von aliphatischen Dicarbonsäuren mit 3 bis 12 Kohlenstoffatomen, den Säureanhydriden von aliphatischen Monocarbonsäuren mit 2 bis 12 Kohlenstoffatomen und den Säureanhydriden von aliphatischen Dicarbonsäuren mit 4 bis 12 Kohlenstoffatomen.

5. Verfahren nach einem der vorhergehenden Ansprüche, wobei man die Umsetzung in Schritt a) in einem nichtwässrigen Reaktionsmedium durchführt.

6. Verfahren nach einem der Ansprüche 1 bis 4, wobei man die Umsetzung in Schritt a) in Substanz durchführt.

7. Verfahren nach einem der vorhergehenden Ansprüche, wobei man in Schritt a) 1 bis 1,5 Moläquivalente des Acylierungsmittels, bezogen auf den Gehalt an Alkohol I-R im Enantiomerengemisch, einsetzt.

8. Verfahren nach einem der vorhergehenden Ansprüche, wobei das in Schritt a) eingesetzte Enantiomerengemisch das Racemat der Alkohole I-S und I-R ist.

9. Verfahren nach einem der vorhergehenden Ansprüche, bei dem man das in Schritt a) eingesetzte Enantiomerengemisch der Alkohole I-R und I-S durch Umsetzung von Thiophen-2-carbaldehyd mit Acetonitril in Gegenwart einer Base gewinnt.

## Claims

1. A process for the preparation of enantiomerically pure (S)-3-methylamino-1-(thien-2-yl)propan-1-ol of the formula II-S 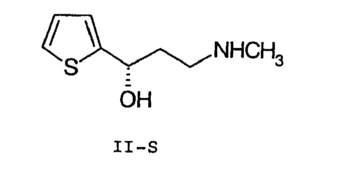 comprising the following steps:
a) reaction of an enantiomer mixture of the alcohols (S)-3-hydroxy-3-thien-2-ylpropionitrile and (R)-3-hydroxy-3-thien-2-ylpropionitrile of the formulae I-S and I-R 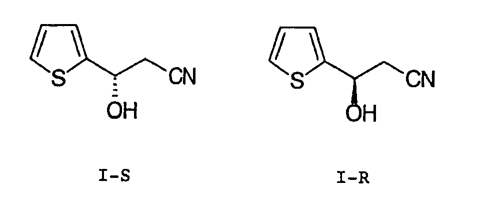 with an acylating agent in the presence of a lipase, a mixture of essentially unacylated alcohol I-S and essentially acylated alcohol I-R being obtained;
b) separation of the alcohol I-S from the mixture obtained in step a); and
c) reaction of the alcohol I-S with hydrogen and methylamine in the presence of a catalyst to give (S)-3-methylamino-1-(thien-2-yl)propan-1-ol II-S.

2. The process according to claim 1, where the lipase in step a) is selected from among lipases from bacteria of the genera Burkholderia or Pseudomonas.

3. The process according to claim 2, where the lipase is a lipase from Burkholderia plantarii, Burkholderia cepacia, Burkholderia glumae, Pseudomonas aeruginosa, Pseudomonas fluorescens, Pseudomonas fragi, Pseudomonas luteola, Pseudomonas vulgaris, Pseudomonas wisconsinensis and Pseudomonas spec. DSM 8246.

4. The process according to one of the preceding claims, the acylating agent being selected from the vinyl, propenyl or isopropenyl esters of aliphatic monocarboxylic acids having 3 to 12 carbon atoms and of aliphatic dicarboxylic acids having 3 to 12 carbon atoms, the acid anhydrides of aliphatic monocarboxylic acids having 2 to 12 carbon atoms and the acid anhydrides of aliphatic dicarboxylic acids having 4 to 12 carbon atoms.

5. The process according to one of the preceding claims, the reaction in step a) being carried out in a nonaqueous reaction medium.

6. The process according to one of claims 1 to 4, the reaction in step a) being carried out in substance.

7. The process according to one of the preceding claims, 1 to 1.5 mol equivalents of the acylating agent, based on the content of alcohol I-R in the enantiomer mixture, being employed in step a).

8. The process according to one of the preceding claims, the enantiomer mixture employed in step a) being the racemate of the alcohols I-S and I-R.

9. The process according to one of the preceding claims, in which the enantiomer mixture of the alcohols I-R and I-S employed in step a) is obtained by reaction of thiophene-2-carbaldehyde with acetonitrile in the presence of a base.

## Revendications

1. Procédé de préparation de (S)-3-méthylamino-1-(thién-2-yl)propan-1-ol à l'état d'énantiomère pur de la formule II-S : 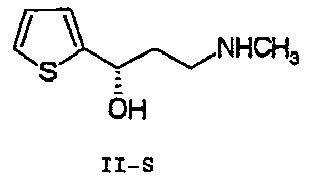 comprenant les étapes suivantes :
a) une réaction d'un mélange d'énantiomères des alcools (S)-3-hydroxy-3-thién-2-ylpropionitrile et (R)-3-hydroxy-3-thién-2-ylpropionitrile des formules I-S et I-R : 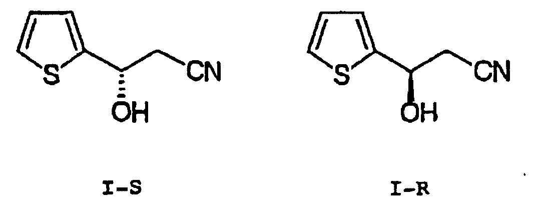 avec un agent d'acylation en présence d'une lipase, avec obtention d'un mélange d'alcool I-S sensiblement non acylé et d'alcool I-R sensiblement acylé,
b) un isolement de l'alcool I-S à partir du mélange obtenu dans l'étape a) et,
c) une réaction de l'alcool I-S avec de l'hydrogène et de la méthylamine en présence d'un catalyseur pour former du (S)-3-méthylamino-1-(thién-2-yl)propan-1-ol de formule II-S.

2. Procédé suivant la revendication 1, dans lequel la lipase de l'étape a) est choisie parmi des lipases issues de bactéries des genres Burkholderia ou Pseudomonas.

3. Procédé suivant la revendication 2, dans lequel la lipase est une lipase de Burkholderia plantarii, Burkholderia cepacia, Burkholderia glumae, Pseudomonas aeruginosa, Pseudomonas fluorescens, Pseudomonas fragi, Pseudomonas luteola, Pseudomonas vulgaris, Pseudomonas wisconsinensis et Pseudomonas spec. DSM 8246.

4. Procédé suivant l'une des revendications précédentes, dans lequel l'agent d'acylation est choisi parmi les esters vinyliques, propényliques ou isopropényliques d'acides monocarboxyliques aliphatiques comportant 3 à 12 atomes de carbone et d'acides dicarboxyliques aliphatiques comportant 3 à 12 atomes de carbone, les anhydrides d'acides monocarboxyliques aliphatiques comportant 2 à 12 atomes de carbone et les anhydrides d'acides dicarboxyliques aliphatiques comportant 4 à 12 atomes de carbone.

5. Procédé suivant l'une des revendications précédentes, dans lequel on effectue la réaction de l'étape a) dans un milieu réactionnel non aqueux.

6. Procédé suivant l'une des revendications 1 à 4, dans lequel on effectue la réaction de l'étape a) dans la masse.

7. Procédé suivant l'une des revendications précédentes, dans lequel on met en oeuvre dans l'étape a) 1 à 1,5 équivalent molaire de l'agent d'acylation, par rapport à la teneur en alcool I-R dans le mélange d'énantiomères.

8. Procédé suivant l'une des revendications précédentes, dans lequel le mélange d'énantiomères mis en oeuvre dans l'étape a) est le racémate des alcools I-S et I-R.

9. Procédé suivant l'une des revendications précédentes, dans lequel on produit le mélange d'énantiomères, mis en oeuvre dans l'étape a), des alcools I-R et I-S par réaction de thiophène-2-carbaldéhyde avec de l'acétonitrile en présence d'une base.
